# FASCICULE DE BREVET EUROPEEN

(11) **EP 3 727 413 B1**
(45) Date de publication et mention de la délivrance du brevet: **03.01.2024**
(21) Numéro de dépôt: 18836821.1
(22) Date de dépôt: 20.12.2018
(51) Int. Cl.: A61K 31/07, A61K 31/11, A61K 31/203, A61K 36/28, A61P 17/08, A61P 17/10, A61K 8/67, A61K 8/9789

(54) **ASSOCIATION D'UN RETINOIDE ET D'UN EXTRAIT DE SILYBUM MARIANUM (L.) GAERTN**
KOMBINATION EINES RETINOIDS UND EINES EXTRAKTS VON SILYBUM MARIANUM (L.) GAERTN
COMBINATION OF A RETINOID AND AN EXTRACT OF SILYBUM MARIANUM (L.) GAERTN

(30) Priorité: 20.12.2017 FR 1762606
(43) Date de publication de la demande: 28.10.2020
(73) Titulaire: Pierre Fabre Dermo-Cosmétique, 81500 Lavaur (FR)
(72) Inventeur: SAURAT, Jean-Hilaire, 1206 GENEVE (CH); SORG, Olivier, 1202 GENEVE (CH)
(74) Mandataire: Regimbeau
(86) Numéro de dépôt international: PCT/EP2018/086326
(87) Numéro de publication internationale: WO 2019/122203

(56) Documents cités:
- WO-A1-2017/037534
- HAN G ET AL: "Composition useful as external skin composition and cosmetics composition for preventing atopic dermatitis and suppressing skin rash, comprises milk thistle extract", WPI / THOMSON,, vol. 2014, no. 8, 8 juillet 2010 (2010-07-08), XP002732291,

## Description

### DOMAINE TECHNIQUE

La présente invention concerne une association d'un rétinoïde, à savoir le rétinaldéhyde, et d'un extrait de *Silybum marianum* (L.) Gaertn., ainsi qu'une composition dermatologique ou dermo-cosmétique, de préférence topique, contenant une telle association, notamment pour le traitement de l'acné, de la séborrhée, de la rosacée et/ou de la dermite séborrhéique.

### ETAT DE LA TECHNIQUE ANTERIEURE

Le nom scientifique *Silybum marianum* (L.) Gaertn. désigne une plante appartenant à la famille des Asteraceae, annuelle ou bisannuelle à tige robuste pouvant atteindre plus de un mètre de hauteur. Ses grandes feuilles luisantes, alternées, sans stipule sont marbrées de blanc et bordées d'épines dures et pointues. Les fleurs sont groupées en capitules terminaux, souvent solitaires. Ils sont entourés de grandes bractées épineuses à l'extrémité très acérée. Les fleurs, tubulées, à cinq lobes, sont de couleur pourpre violacé. Les fruits sont des akènes luisants, noirs ou marbrés de jaune, surmontés d'une aigrette à soies denticulées en anneau à leur base. Le nom vernaculaire de cette plante est Chardon-Marie.

L'akène (souvent dénommé graine de manière erronée dans la littérature) de *Silybum marianum* (L.) Gaertn. et ses préparations sont traditionnellement utilisés par voie orale, dans le traitement symptomatique des troubles fonctionnels digestifs attribués à une origine hépatique.

Dans la littérature, le principe actif principal de l'akène de *Silybum marianum* (L.) Gaertn. est la silymarine, mélange de plusieurs flavonolignanes (principalement silybine, isosilybine, silychristine et silydianine). Les akènes contiennent jusqu'à 3% en poids de silymarine. Ils sont également constitués d'huile (20-30% en poids), de mucilages et de protéines.

Par ailleurs, un déficit en vitamine A a été démontré dans la peau des patients acnéiques (Röllman, O., and Vahlquist, A. (1985). Vitamin A in skin and serum-studies of acné vulgaris, atopic dermatitis, ichthyosis vulgaris and lichen planus. Br. J. Dermatol. 113(4), 405-413) et une déficience de son métabolisme a été décrit dans l'épithélium pilosébacé (Everts, H. B. (2012). Endogenous retinoids in the hair follicle and sebaceous gland. Biochim. Biophys. Acta 1821(1), 222-229). Le rétinaldéhyde en application topique permet de délivrer les différents composants de la Vitamine A dans la peau humaine (Sorg, O., and Saurat, J. H. (2014). Topical retinoids in skin ageing : A focused update with référencé to sun induced epidermal vitamin A deficiency. Dermatology 228(4), 314-325), propriété qui est déjà utilisée dans la prévention et le traitement des peaux à tendance acnéique.

La demande WO2017/037534 décrit en outre des compositions utiles pour le traitement du psoriasis comprenant de la vitamine A et une huile d'akènes de *Silybum marianum.*

### RESUME DE L'INVENTION

Dans ce contexte, la Demanderesse a mis en évidence de manière surprenante qu'un rétinoïde, à savoir le rétinaldéhyde (encore appelé rétinal), en association avec un extrait de *Silybum marianum,* présentait des propriétés améliorées pour le traitement de l'acné, de la séborrhée, de la rosacée ou de la dermite séborrhéique. Plus particulièrement, les résultats d'études obtenus ont démontré le rôle synergique du rétinaldéhyde associé à un extrait de *Silybum marianum* dans la production d'acides rétinoïdes par les cellules de la peau.

Un objet de l'invention concerne donc une association comprenant un rétinoïde et un extrait de *Silybum marianum* (L.) Gaertn., caractérisée en ce que le rétinoïde est le rétinaldéhyde et l'extrait de *Silybum marianum* (L.) Gaertn. est un extrait d'akènes de *Silybum marianum* (L.) Gaertn. comprenant moins de 0,2% en poids de silymarine par rapport au poids de l'extrait sec.

Un autre objet de l'invention concerne une association comprenant un rétinoïde et un extrait de *Silybum marianum* (L.) Gaertn. telle que définie ci-dessus pour son utilisation comme médicament.

Un autre objet de l'invention concerne une association comprenant un rétinoïde et un extrait de *Silybum marianum* (L.) Gaertn. telle que définie ci-dessus pour son utilisation dans le traitement de l'acné, de la séborrhée, de la rosacée et/ou de la dermite séborrhéique.

Un autre objet de l'invention concerne une composition dermatologique ou dermo-cosmétique, de préférence topique, comprenant une association comprenant un rétinoïde et un extrait de *Silybum marianum* (L.) Gaertn. telle que définie ci-dessus en combinaison avec au moins un excipient dermatologiquement ou dermo-cosmétiquement acceptable.

Un autre objet de l'invention concerne une composition dermatologique ou dermo-cosmétique, de préférence topique, comprenant une association comprenant un rétinoïde et un extrait de *Silybum marianum* (L.) Gaertn. telle que définie ci-dessus en combinaison avec au moins un excipient dermatologiquement ou dermo-cosmétiquement acceptable pour son utilisation dans le traitement de l'acné, de la séborrhée, de la rosacée et/ou de la dermite séborrhéique.

### DESCRIPTION DETAILLEE

### Définitions

Dans la présente description, la plante *Silybum marianum* (L.) Gaertn. pourra être désignée de manière abrégée par le terme *Silybum marianum* et le rétinaldéhyde pourra être désigné de manière abrégé par RAL.

Par « acide gras », on entend, au sens de la présente invention, un acide carboxylique R1CO₂H dont la chaîne R1 est une chaîne hydrocarbonée linéaire ou ramifiée, saturée ou comprenant des doubles liaisons C=C, l'acide carboxylique comprenant de 16 à 22 atomes de carbone (incluant l'atome de carbone de la fonction acide carboxylique).

Par acide gras (incluant l'acide linoléique) « libre », on entend, au sens de la présente invention, un acide gras non lié à d'autres molécules (par ex. à du glycérol ou des dérivés de celui-ci pour donner des glycérides ou à un alcool pour donner un ester gras).

Par « alcool en C₁ en C₃ », on entend, au sens de la présente invention, un alcool R2OH dont la chaîne R2 est une chaîne hydrocarbonée saturée, linéaire ou ramifiée, comprenant 1 à 3 atomes de carbone. Il pourra s'agir du méthanol, de l'éthanol, du *n-*propanol ou de l'isopropanol, en particulier du méthanol, de l'éthanol ou de l'isopropanol. De préférence, il s'agira de l'isopropanol.

Par « dermatologiquement ou dermo-cosmétiquement acceptable », on entend désigner, dans la présente invention, ce qui est utile dans la préparation d'une composition dermatologique ou dermo-cosmétique, qui est généralement sûr, non toxique et ni biologiquement ni autrement non souhaitable et qui est acceptable pour une utilisation thérapeutique ou cosmétique, notamment par application topique.

Par « environ », on entend dans la présente description que la valeur concernée peut être inférieure ou supérieure de 10%, notamment de 5%, en particulier de 1%, à la valeur indiquée.

Par « extrait sec », on entend, au sens de la présente invention, un extrait dépourvu de solvant d'extraction ou en contenant uniquement à l'état de trace non significative. Un tel extrait sec contient ainsi uniquement de la matière issue de *Silybum marianum* (L.) Gaertn.. Il peut contenir également des traces non significatives de solvant d'extraction.

Par « isosilybine », on entend, au sens de la présente invention, les deux diastéréoisomères isosilybine A et isosilybine B.

Par « silybine », encore appelée dans l'art silibinine, on entend, au sens de la présente invention, les quatre diastéréoisomères silybine A, silybine B, 2,3-cis-silybine A et 2,3-cis-silybine B.

Par « silychristine », on entend, au sens de la présente invention, les deux diastéréoisomères silychristine A et silychristine B.

Par « silymarine », on entend au sens de la présente invention un extrait purifié d'akènes de *Silybum marianum* (L.) Gaertn. comprenant majoritairement (au moins 95% en poids) un mélange des quatre flavonolignanes suivants : silybine, isosilybine, silychristine et silydianine (Kuki *et al.* 2012). Une teneur en silymarine inférieure à 0,2% en poids signifie donc que la quantité totale des constituants de la silymarine est inférieure à 0,2% en poids. Une telle teneur peut être déterminée notamment par HPLC (chromatographie en phase liquide à haute performance) ou UPLC (chromatographie en phase liquide à ultra haute performance) en calculant l'aire totale des pics correspondant à tous les constituants de la silymarine, notamment en utilisant un échantillon de silymarine de référence, qui peut être obtenu par exemple auprès de Sigma Aldrich, pour déterminer la position de ces pics.

Par « solvant organique non miscible à l'huile issue d'akènes de *Silybum marianum* (L.) Gaertn. », on entend, au sens de la présente invention, un solvant organique qui n'est pas capable de se mélanger, ou seulement partiellement, avec l'huile issue d'akènes de *Silybum marianum* (L.) Gaertn., de sorte que le mélange du solvant organique et de l'huile issue d'akènes de *Silybum marianum* (L.) Gaertn. donne un mélange hétérogène dans lequel il peut être observé au moins deux phases distinctes.

Par « stérol », on entend, au sens de la présente invention, une molécule possédant un noyau de stérane dont le carbone 3 est porteur d'un groupe hydroxyle OH, le stérane ayant la structure suivante :

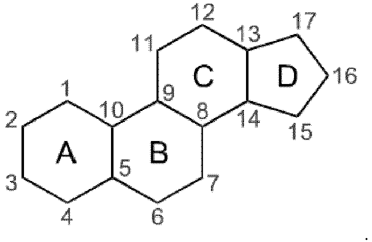

Par « tocophérol », on entend, au sens de la présente invention, l'α-tocophérol, le β-tocophérol, le γ-tocophérol et le δ-tocophérol.

### Rétinoïde

Le rétinoïde utilisé dans le cadre de la présente invention est le rétinaldéhyde.

### Extrait de Silybum marianum (L.) Gaertn.

L'extrait utilisé dans le cadre de la présente invention est un extrait de *Silybum marianum,* et notamment un extrait d'akènes de *Silybum marianum.*

Selon un mode de réalisation particulier, l'extrait selon l'invention contient moins de 0,2%, de préférence moins de 0,1% en poids de silymarine par rapport au poids de l'extrait sec.

Selon un mode de réalisation particulier, l'extrait selon l'invention contient au moins 0,5% en poids, de préférence au moins 1,0% en poids de béta-sitostérol par rapport au poids de l'extrait sec. En particulier, l'extrait selon l'invention contient entre 0,5% et 2,5% en poids, notamment entre 1,0% et 2,0% en poids, par exemple environ 1,5% en poids de béta-sitostérol par rapport au poids de l'extrait sec. Avantageusement, l'extrait selon l'invention contient moins de 0,2%, de préférence moins de 0,1% en poids de silymarine par rapport au poids de l'extrait sec. Le rapport massique silymarine/béta-sitostérol de l'extrait selon l'invention pourra être en particulier inférieur à 0,4, notamment inférieur à 0,07. L'extrait selon l'invention pourra comprendre par ailleurs entre 2 et 7% en poids, notamment entre 3 et 6% en poids, par exemple entre 3 et 5% en poids de stérols par rapport au poids de l'extrait sec.

Selon un mode de réalisation particulier, l'extrait selon l'invention contient au moins 3% en poids, de préférence au moins 4% en poids d'acide linoléique libre par rapport au poids de l'extrait sec. En particulier, l'extrait selon l'invention contient entre 3% et 15% en poids, notamment entre 4% et 10% en poids, en particulier entre 4% et 6% en poids, par exemple environ 5% en poids d'acide linoléique libre par rapport au poids de l'extrait sec. Avantageusement, l'extrait selon l'invention contient moins de 0,2%, de préférence moins de 0,1% en poids de silymarine par rapport au poids de l'extrait sec. L'extrait selon l'invention pourra comprendre par ailleurs entre 10% et 70%, en particulier entre 10% et 30% en poids, notamment entre 15% et 25% en poids d'acides gras libres par rapport au poids de l'extrait sec.

Selon un autre mode de réalisation particulier, l'extrait selon l'invention contient entre 0,5% et 2,5% en poids, notamment entre 1,0% et 2,0% en poids, par exemple environ 1,5% en poids de béta-sitostérol par rapport au poids de l'extrait sec et entre 3% et 15% en poids, notamment entre 4% et 10% en poids, en particulier entre 4% et 6% en poids, par exemple environ 5% en poids d'acide linoléique libre par rapport au poids de l'extrait sec. Avantageusement, l'extrait selon l'invention contient moins de 0,2%, de préférence moins de 0,1% en poids de silymarine par rapport au poids de l'extrait sec. Le rapport massique silymarine/béta-sitostérol de l'extrait selon l'invention pourra être en particulier inférieur à 0,4, notamment inférieur à 0,07. L'extrait selon l'invention pourra comprendre par ailleurs entre 2 et 7% en poids, notamment entre 3 et 6% en poids, par exemple entre 3 et 5% en poids de stérols par rapport au poids de l'extrait sec et entre 10% et 50%, en particulier entre 10% et 30% en poids, notamment entre 15% et 25% en poids d'acides gras libres par rapport au poids de l'extrait sec.

Selon un mode de réalisation particulier, l'extrait selon l'invention contient au moins 0,01% en poids, notamment au moins 0,05% en poids de tocophérols par rapport au poids de l'extrait sec. En particulier, l'extrait selon l'invention contient entre 0,01% et 0,5% en poids, notamment entre 0,05% et 0,2% en poids, par exemple environ 0,1% en poids de tocophérols par rapport au poids de l'extrait sec. Avantageusement, l'extrait selon l'invention contient moins de 0,2%, de préférence moins de 0,1% en poids de silymarine par rapport au poids de l'extrait sec. Le rapport massique silymarine/tocophérols de l'extrait selon l'invention pourra être en particulier inférieur à 1, notamment inférieur à 0,1.

Selon un autre mode de réalisation particulier, l'extrait selon l'invention contient moins de 0,2%, de préférence moins de 0,1% en poids de silymarine par rapport au poids de l'extrait sec ; entre 0,5% et 2,5% en poids, notamment entre 1,0% et 2,0% en poids, par exemple environ 1,5% en poids de béta-sitostérol par rapport au poids de l'extrait sec ; entre 3% et 15% en poids, notamment entre 4% et 10% en poids, en particulier entre 4% et 6% en poids, par exemple environ 5% en poids d'acide linoléique libre par rapport au poids de l'extrait sec ; et entre 0,01% et 0,5% en poids, notamment entre 0,05% et 0,2% en poids, par exemple environ 0,1% en poids de tocophérols par rapport au poids de l'extrait sec. L'extrait selon l'invention pourra comprendre par ailleurs entre 2 et 7% en poids, notamment entre 3 et 6% en poids, par exemple entre 3 et 5% en poids de stérols par rapport au poids de l'extrait sec et entre 10% et 50%, en particulier entre 10% et 30% en poids, notamment entre 15% et 25% en poids d'acides gras libres par rapport au poids de l'extrait sec. Le rapport massique silymarine/béta-sitostérol de l'extrait selon l'invention pourra être en particulier inférieur à 0,4, notamment inférieur à 0,07. Le rapport massique silymarine/tocophérols de l'extrait selon l'invention pourra être en particulier inférieur à 1, notamment inférieur à 0,1.

De préférence, l'extrait selon la présente invention sera un extrait sec.

L'extrait selon la présente invention pourra être obtenu par extraction d'une huile issue d'akènes de *Silybum marianum* (L.) Gaertn. par un solvant d'extraction comprenant, notamment constitué par, une solution aqueuse hydrotropique, de l'eau subcritique ou un solvant organique non miscible à l'huile issue d'akènes de *Silybum marianum* (L.) Gaertn. éventuellement en mélange avec de l'eau ; en particulier un solvant organique non miscible à l'huile issue d'akènes de *Silybum marianum* (L.) Gaertn., tel qu'un un alcool en C₁ en C₃, éventuellement en mélange avec de l'eau.

En particulier, le solvant d'extraction comprendra, notamment sera constitué par, le méthanol, l'éthanol ou l'isopropanol, éventuellement en mélange avec de l'eau, notamment dans un rapport volumique solvant organique/eau compris entre 80/20 et 100/0, notamment compris entre 85/15 et 95/5, en particulier d'environ 90/10. Avantageusement, il s'agira d'un mélange isopropanol / eau, notamment dans un rapport volumique d'environ 90/10.

L'huile issue d'akènes de *Silybum marianum* peut être avantageusement obtenue par extraction à partir d'akènes de *Silybum marianum* (L.) Gaertn. (les akènes pourront être entiers ou en morceaux), notamment par pression, avantageusement par pression à froid (c'est-à-dire sans chauffage, à température ambiante).

L'étape d'extraction de l'huile issue d'akènes de *Silybum marianum* peut être effectuée par mélange de l'huile avec le solvant d'extraction durant 1 à 12 h et en particulier à une température comprise entre 15 et 25°C, notamment d'environ 20°C. La quantité de solvant d'extraction utilisé pour effectuer cette extraction sera avantageusement de 0,5 à 3 g, en particulier de 1 à 3 g pour 1 g d'huile.

La phase d'extraction ainsi obtenue est ensuite séparée de la phase lipidique, avant d'être séchée et évaporée, partiellement ou totalement, notamment sous vide, pour éliminer plus ou moins le solvant d'extraction et obtenir soit l'extrait sec si on élimine totalement le solvant, soit l'extrait concentré qui est dilué dans du solvant résiduel.

### Association selon l'invention

L'association selon l'invention comprend un rétinoïde tel que défini ci-dessus et et un extrait de *Silybum marianum* (L.) Gaertn. tel que défini ci-dessus.

### Compositions dermatologiques et dermo-cosmétiques selon l'invention

La présente invention a également pour objet une composition dermatologique ou dermo-cosmétique comprenant au moins une association telle que définie ci-dessus en combinaison avec au moins un excipient dermatologiquement ou dermo-cosmétiquement acceptable, plus particulièrement destinée à une application topique, notamment sur la peau.

Les compositions dermatologiques et dermo-cosmétiques selon l'invention pourront se présenter sous les formes qui sont habituellement connues pour une administration topique, c'est à dire notamment les lotions, les mousses, les gels, les dispersions, les émulsions, les sprays, les sérums, les masques ou les crèmes, avec des excipients permettant notamment une pénétration cutanée afin d'améliorer les propriétés et l'accessibilité du principe actif. Avantageusement, il s'agira d'une crème.

Ces compositions contiennent généralement, outre les ingrédients de l'association selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Ces compositions peuvent en outre contenir d'autres principes actifs conduisant à un effet complémentaire ou éventuellement synergique.

Avantageusement, les compositions selon la présente invention comprendront 0,01 à 10% en poids, de préférence 0,05 à 5% en poids, d'un extrait de *Silybum marianum* selon l'invention (de préférence en poids d'extrait sec) par rapport au poids total de la composition.

Avantageusement, les compositions selon la présente invention comprendront 0,001 à 5% en poids, de préférence 0,01 à 1% en poids, du rétinoïde par rapport au poids total de la composition.

### Applications thérapeutiques

Les associations selon l'invention et les compositions dermatologiques ou dermo-cosmétiques selon l'invention sont utiles dans le traitement de l'acné (par ex. acné juvénile, encore appelée acné de l'adolescent, ou acné tardive, pouvant être kystique), de la séborrhée, de la dermite séborrhéique et/ou de la rosacée, de préférence par application topique, en particulier sur la peau.

La présente invention est illustrée par les exemples non limitatifs qui suivent.

### FIGURES

La Figure 1 représente le chromatogramme UPLC de l'extrait I de *Silybum marianum* obtenu selon le Protocole 1.
Les Figures 2A, 2B et 2C représentent respectivement un chromatogramme UPLC de l'extrait M, de l'extrait E et de l'extrait I de *Silybum marianum* obtenu selon le Protocole 2.
Les Figures 3A, 3B et 3C représentent respectivement la zone 10-22 min (correspondant à la zone des acides gras) d'un chromatogramme GC / MS de l'extrait E, de l'extrait M et de l'extrait I de *Silybum marianum* obtenu selon le Protocole 3.

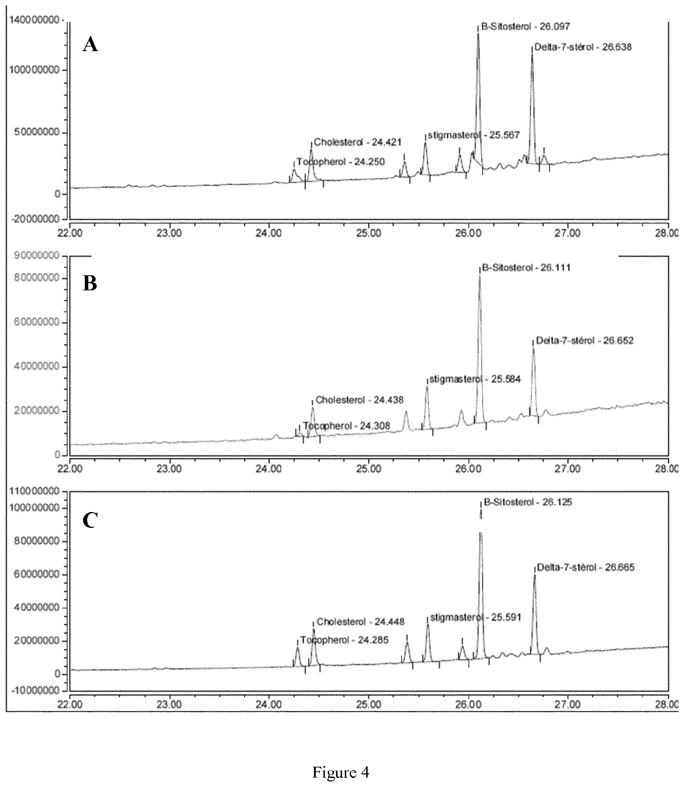

Les Figures 4A, 4B et 4C représentent respectivement la zone 22-28 min (correspondant à la zone des stérols) d'un chromatogramme GC / MS de l'extrait E, de l'extrait M et de l'extrait I de *Silybum marianum* obtenu selon le Protocole 3.
La Figure 5 représente la quantité produite par les cellules de la peau de rétinoïdes totaux en fonction du traitement reçu, à savoir le véhicule seul (EtOH), du rétinaldéhyde à 2µM (RAL 2µM), un extrait de *Silybum marianum* à 50 µg/mL (SYL 50 µg/mL) ou l'association du rétinaldéhyde à 2µM et de l'extrait de *Silybum marianum* à 50 µg/mL (RAL + SYL).
La Figure 6 représente la quantité produite par les cellules de la peau de rétinoïdes totaux en fonction du traitement reçu, à savoir le véhicule seul (EtOH), du rétinol à 2µM (ROL 2µM), un extrait de *Silybum marianum* à 50 µg/mL (SYL 50 µg/mL) ou l'association du rétinol à 2µM et de l'extrait de *Silybum marianum* à 50 µg/mL (ROL + SYL).

### EXEMPLES

### I - Exemples de préparations d'extraits selon l'invention

Extrait isopropanolique 90 **(extrait I)** obtenu selon le procédé suivant :
- pression à froid d'akènes de *Silybum marianum* pour obtenir une huile issue d'akènes de *Silybum marianum,*
- extraction de l'huile issue d'akènes de *Silybum marianum* par un mélange isopropanol/eau (90/10 v/v) avec 1 gramme du mélange isopropanol / eau par gramme d'huile pendant 2 heures à 20°C,
- récupération de la phase isopropanolique, et
- évaporation du solvant.

Des extraits méthanolique **(extrait M)** et éthanolique 90 **(extrait E)** ont été obtenus de manière similaire en remplaçant le mélange isopropanol/eau (90/10 v/v) respectivement par du méthanol et un mélange éthanol/eau (90/10 v/v). L'extraction de l'huile issue d'akènes de *Silybum marianum* se fait respectivement par 3 volumes de méthanol et par 3 volumes du mélange éthanol/eau (90/10 v/v) pour 1 volume d'huile pendant 2 heures à 20°C.

Ces différents extraits ont été caractérisés par UPLC (chromatographie en phase liquide à ultra haute performance) ou par GC-MS (chromatographie en phase gazeuse couplée à la spectrométrie de masse) selon les protocoles détaillés ci-dessous.

### Protocoles d'évaluation des extraits obtenus :

### 1. Protocole 1 : Evaluation de la teneur en Silymarine par UPLC

### ∘ Préparation de l'échantillon et du témoin:

- Témoin silymarine : Préparer une solution de silymarine à 5 mg dans 10 ml d'un mélange méthanol / eau (60:40) (v/v).
- Echantillon :
   - Extrait A : Préparer une solution à 100 mg d'extrait à analyser dans 10 ml d'un mélange méthanol / dichlorométhane (70:30) (v/v)
   - Extraits M, E, I : Chauffer l'extrait sec à analyser à 35°C sous agitation jusqu'à obtention d'une solution homogène et limpide. Peser exactement 200 mg (*pe*) de l'extrait, le solubiliser dans 10 ml d'un mélange méthanol / dichlorométhane permettant la solubilisation totale de l'extrait et homogénéiser la solution. Ce mélange va du ratio méthanol / dichlorométhane (1:1) (v/v) au méthanol pur.

### ∘ Conditions analytiques :

- *Colonne* : Acquity BEH Shield C18 150 mm x 2,1 mm - 1,7µm (Waters)
- *Phase mobile :*
∘ A : Eau + 0,1% d'acide formique
∘ B : Acétonitrile + 0,1% d'acide formique

- *Gradient* :

| **T (min)** | **A (%)** | **B (%)** |
|---|---|---|
| 0 | 90 | 10 |
| 15 | 60 | 40 |
| 20 | 0 | 100 |
| 39,5 | 0 | 100 |
| 40 | 90 | 10 |
| 45 | 90 | 10 |

- *Température de la colonne* : 40°C
- *Débit* : 0,4 ml/min
- *Détection* : 287 nm
- *Volume d'injection* : 1 µL

### 2. Protocole 2 : Evaluation de la teneur en Acide linoléique par UPLC

### ∘ Préparation de l'échantillon et du témoin

- Témoin Acide linoléique : Préparer une solution d'acide linoléique à 10 mg dans 10 ml d'un mélange méthanol / dichlorométhane (1: 1) (v/v).
- Echantillon :
   - Extraits M, E, I : Chauffer l'extrait sec à analyser à 35°C sous agitation jusqu'à obtention d'une solution homogène et limpide. Peser exactement 50 mg (*pe*) de l'extrait, le solubiliser dans 1 ml d'un mélange méthanol / dichlorométhane permettant la solubilisation totale de l'extrait et homogénéiser la solution. Ce mélange va du ratio méthanol / dichlorométhane (1: 1) (v/v) au méthanol pur.

### ∘ Conditions analytiques

- *Colonne* : Acquity BEH Shield C18 150 mm x 2,1 mm - 1,7µm (Waters)
- *Phase mobile :*
∘ A : Eau + 0,1% d'acide formique
∘ B : Acétonitrile + 0,1% d'acide formique

- *Gradient :*

| **T (min)** | **A (%)** | **B (%)** |
|---|---|---|
| 0 | 50 | 50 |
| 1 | 50 | 50 |
| 10 | 0 | 100 |
| 15 | 0 | 100 |
| 15,5 | 50 | 50 |
| 20 | 50 | 50 |

- *Température de la colonne* : 40°C
- *Débit* : 0,4 ml/min
- *Détection* : 215 nm
- *Volume d'injection* : 1 µL

### 3. Protocole 3 : Evaluation de la teneur en Acides gras et Stérols par GC-MS

### ∘ Préparation de l'échantillon

- Chauffer l'extrait sec à analyser à 35°C en agitant jusqu'à obtention d'un liquide homogène limpide
- Solubiliser 20 mg de l'extrait dans 800 µL d'un mélange méthanol / dichlorométhane (1: 1) (v/v)
- Ajouter 200 µL du dérivatisant *N*,*O*-Bis(triméthylsilyl)trifluoroacétamide (BSTFA) + Triméthylchlorosilane (TMCS) (99:1) (Supelco - Sigma Aldrich)
- Agiter 1 minute au Vortex

### ∘ Conditions de la chromatographie en phase gazeuse (CPG)

- *Colonne* : DB-5ms (Agilent technologies) ; 30 m x 0,25 mm ; 0,25 µm
- *Injection* : T = 300°C ; Mode = Split ; Split ratio = 100 : 1
- *Four* : Gradient de température (°C) :
   ∘ Température initiale = 150°C
   ∘ Gradient = 7°C / min jusqu'à Température finale = 340°C
   ∘ Maintenir à 340°C pendant 10 minutes
- *Débit gaz vecteur* : 1 mL/min
- *Détection* : MS - EI; T = 300°C ; Scan Time = 0,2 sec. ; Full Scan Start Mass = 40 ; Full Scan End Mass = 600
- *Volume d'injection* : 1µL

### Résultats :

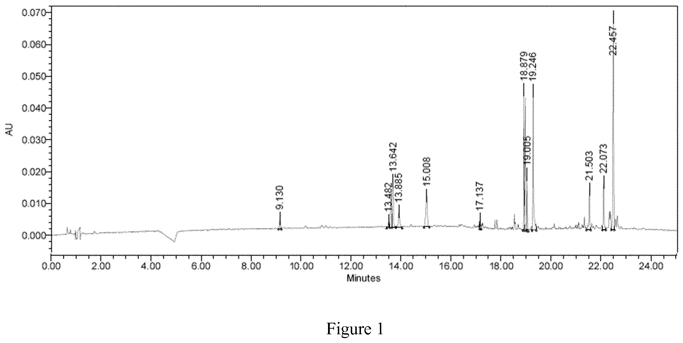

L'extrait I contient majoritairement des substances ayant un temps de rétention entre 13 et 30 minutes par UPLC (cf. Figure 1). Il présente les caractéristiques suivantes :

| **Composants** | | **% en poids** |
|---|---|---|
| Silymarine | | 0,06 |
| Acides gras libres (principalement acides palmitique, oléique et linoléique) | | 24,6 |
| | *dont acide linoléique* | *5,1* |
| Stérols | | 3,6 |
| | *dont béta-sistérol* | *1,5* |

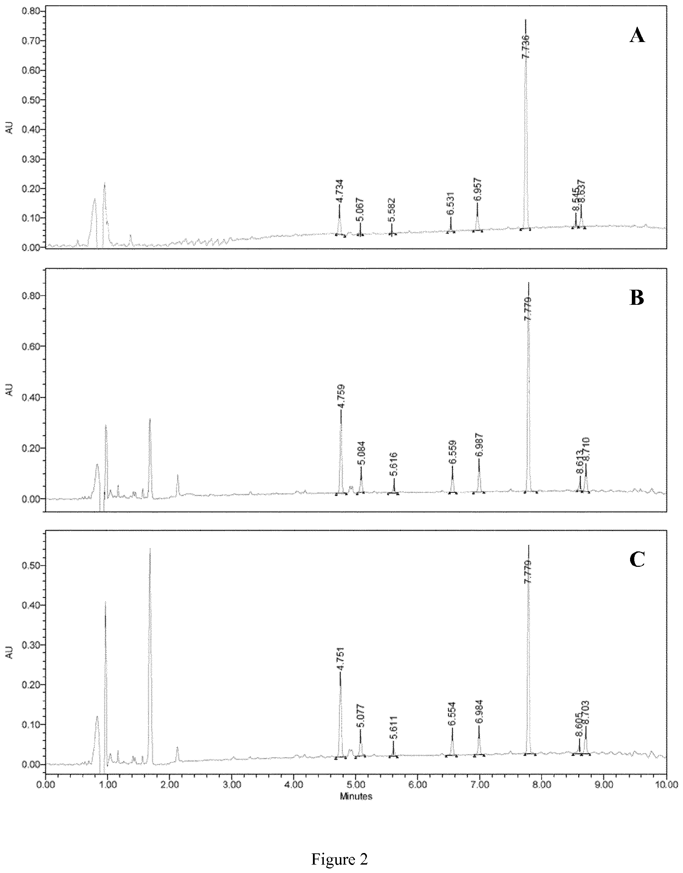

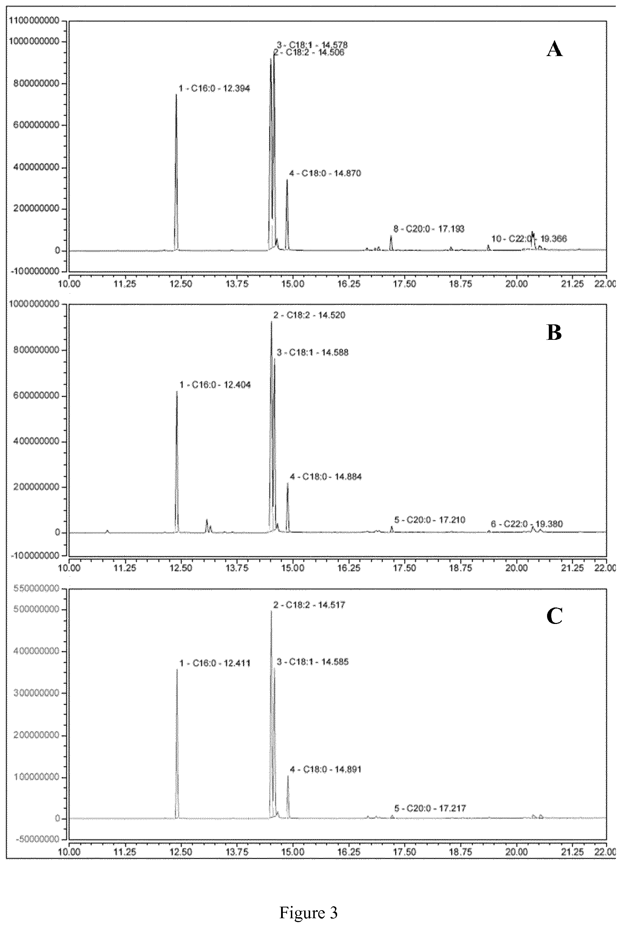

Les chromatogrammes UPLC et GC / MS des 3 extraits (extraits I, M et E) de *Silybum marianum* sont similaires (cf. Figures 2 et 3).

### II - Exemples de compositions comprenant un rétinoïde et/ou un extrait de Silybum marianum selon l'invention

### COMPOSITION 1 (crème) à base de rétinaldéhyde = RAL1

| **INCI désignation** | **Pourcentage en poids** |
|---|---|
| Oléamide de glycylglycine | 0,1% |
| Tocophéryl glucoside | 0,1% |
| rétinaldéhyde | 0,05% |
| Gélifiant Carbomer | 0,35% |
| Squalane | 15% |
| Mineral Oil | |
| Caprylic capric triglycérides | |
| Eau | Qsp 100% |

### COMPOSITION 2 (crème) à base de rétinaldéhyde = RAL2

| **INCI désignation** | **Pourcentage en poids** |
|---|---|
| Eau | QSP 100% |
| Sorbate Potassium | 0,3 |
| Ethanol | 3 |
| Glycolic Acid | 6 |
| Ceteareth 33 & cetearyl alcool | 3 |
| Polysorbate 60 | 6 |
| Cetylic Alccol | 11 |
| Butylhydroxytoluene | 0,01 |
| Cyclopentasiloxane | 10 |
| Hydroxyde Sodium | qs |
| Rétinaldéhyde | 0,1 |
| Undecyl Rhamnoside | 0,2 |

### COMPOSITION 3 (crème) à base de rétinaldéhyde = RAL3

| **INCI désignation** | **Pourcentage en poids** |
|---|---|
| Eau | QSP 100% |
| Glycérine | 6 |
| Disodium EDTA | 0,1 |
| Pentylène glycol | 3 |
| Glyceryl Stéarate & PEG-100 Stéarate | 3 |
| Isododecane | 7 |
| Butylhydroxytoluene | 0,02 |
| Carbomer Papaine Crosspolymer | 1 |
| Rétinaldéhyde | 0,1 |
| Caprylic/Capric Triglycérides | 7 |
| 1α-linolénate de 2-hydroxy-octyle | 0,6 |

### COMPOSITION 4 (crème) à base de rétinaldéhyde = RAL4

Le rétinaldéhyde a été formulé à 0,1% (p/p) dans un mélange comprenant :
Glycérine 5%
Gélifiant 1,5%
Silice 1%
Eau qs.

### COMPOSITION 5 (crème) à base d'extrait de Silybum marianium = SYL1 L'extrait I préparé à l'exemple 1 a été formulé à 7% (p/p) dans un mélange isopropanol / PEG 300 (1:1) (p/p).

### COMPOSITION 6 (crème) à base d'extrait de Silybum marianium = SYL2 (pourcentage p/p)

SYL1 25%
Glycérine 5%
Gélifiant 1,5%
Silice 1%
Eau qs.

### COMPOSITION 7 (crème) comprenant l'association d'un extrait de Silybum marianum et de rétinaldéhyde = ASSO1 (pourcentage p/p)

SYL1 25%
Rétinaldéhyde 0,1%
Glycérine 5%
Gélifiant 1,5%
Silice 1%
Eau qs.

### COMPOSITION 8 (crème) comprenant l'association d'un extrait de Silybum marianum et de rétinaldéhyde = ASSO2 (Pourcentage p/p)

SYL1 12,5%
Rétinaldéhyde 0,1%
Glycérine 5%
Gélifiant 1,5%
Silice 1%
Eau qs.

### III - Etudes cliniques

Les différentes crèmes testées ont été appliquées sur le visage (peau nettoyée), 2 applications/jour matin et soir par massage doux sauf pour les compositions comprenant du RAL : le soir uniquement.

L'analyse des effets thérapeutiques obtenus a été effectuée selon la méthode d'analyse globale de l'état clinique IGA (Investigator Global Assessment) acceptée par la FDA (Food and Drug Administration) - pour l'acné et adaptée à la rosacée et à la dermite séborrhéique - et appliquée par un dermatologiste expert clinique (Guidance for Industry Acné Vulgaris: Developing Drugs for Treatment).

Avec une telle méthode les Grades IGA suivants sont attribués selon la sévérité de l'acné observée :

| Grade IGA | Etat du patient |
|---|---|
| 0 | Peau nette sans lésions inflammatoires ou non inflammatoires |
| 1 | Peau quasiment nette avec de rares lésions non inflammatoires et pas plus d'une petite lésion inflammatoire |
| 2 | Acné légère de sévérité supérieure au grade 1 : quelques lésions non inflammatoires avec pas plus de quelques lésions inflammatoires (papules et pustules, pas de lésion nodulaire) |
| 3 | Acné modérée de sévérité supérieure au grade 2 : jusqu'à de nombreuses lésions non inflammatoires et potentiellement quelques lésions inflammatoires mais pas plus d'une petite lésion nodulaire |
| 4 | Acné sévère de sévérité supérieure au grade 3 : jusqu'à de nombreuses lésions non inflammatoires et inflammatoires mais pas plus de quelques lésions nodulaires |

Les résultats des différentes analyses sont présentés ci-dessous.

### Analyse 1 : Avant traitement versus Extrait de Silybum marianum sans RAL versus association RAL + Extrait de Silybum marianum

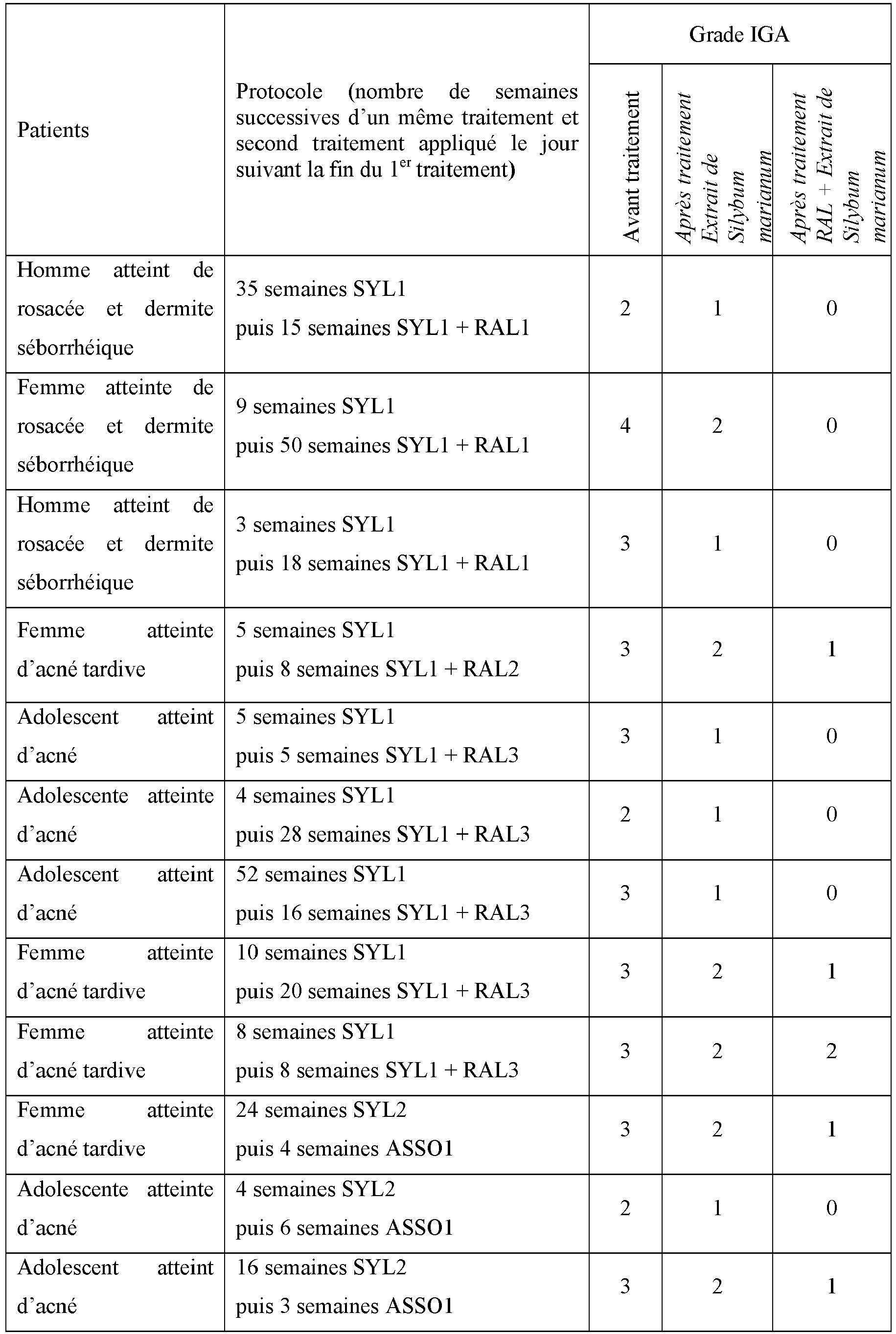

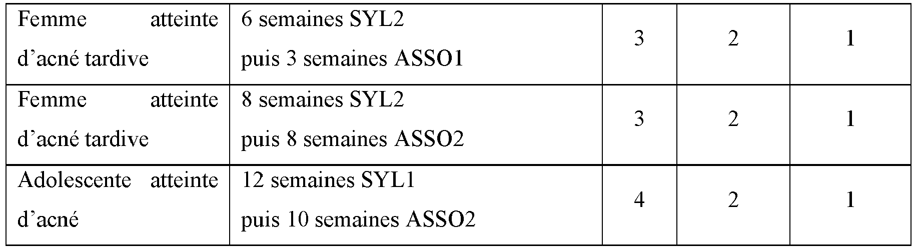

### Analyse 2 : Avant traitement versus RAL sans Extrait de Silybum marianum versus association RAL + Extrait de Silybum marianum

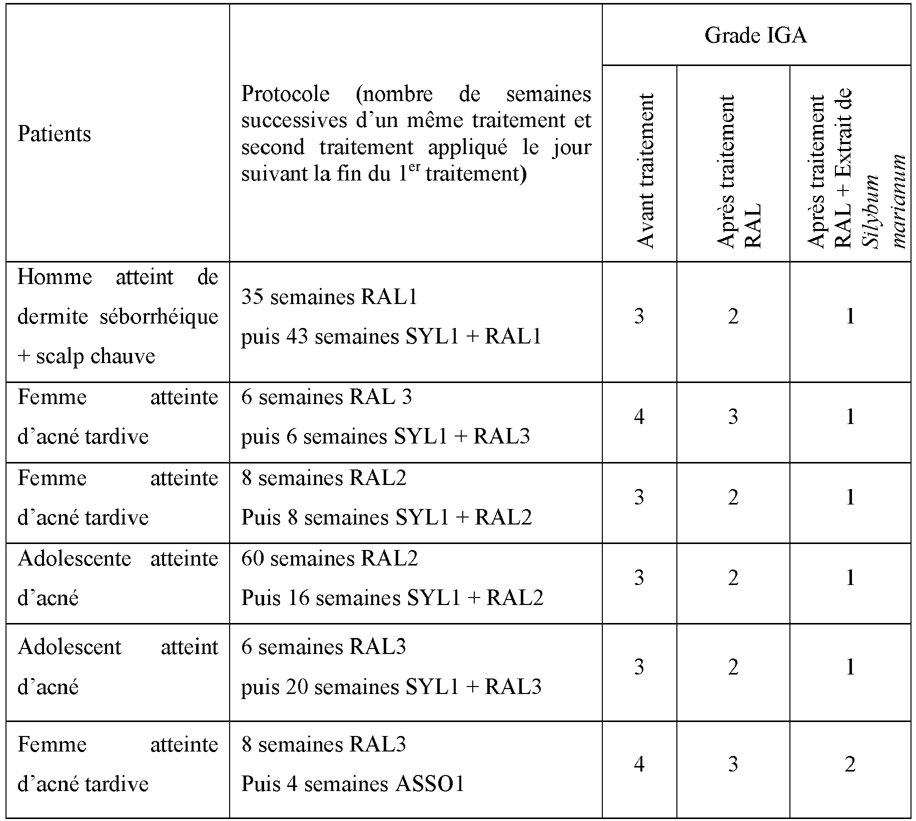

### Conclusion

Les résultats obtenus avec les différents traitements à base respectivement d'une association rétinaldéhyde / extrait de *Silybum marianum,* du rétinaldéhyde seul ou de l'extrait de *Silybum marianum* seul, dans les études cliniques décrites ci-dessus, montrent que le rétinaldéhyde en association avec l'extrait de *Silybum marianum,* permet d'obtenir un meilleur résultat clinique.

### IV - Etudes in vitro : évaluation de la correction du déficit en vitamine A grâce à l'association rétinaldéhyde (RAL) + extrait de Silybum marianum (SYL) selon l'invention ou l'association comparative rétinol (ROL) + extrait de Silybum marianum (SYL)

### Matériel et méthodes :

Culture :
- 3 jours avant l'expérience, cultiver les cellules A431 de carcinome épidermoïde dans 2 plaques à 6 puits à une densité de 40'000 cellules par puits (2 ml par puits).

Traitement des cellules (3 puits par condition, 2 ml par puits ; concentrations finales) :
- Ethanol (solvant) : éthanol 1% ;
- ROL : ROL 10 µM dans éthanol 1% ;
- RAL : RAL 2 µM dans éthanol 1% ;
- SYL : Extrait I de *Silybum marianum* 50 µg/ml dans éthanol 1% ;
- ROL + SYL : ROL 10 µM + Extrait de *Silybum marianum* 50 µg/ml dans éthanol 1% ;
- RAL + SYL : RAL 2 µM + Extrait de *Silybum marianum* 50 µg/ml dans éthanol 1%.

### Incubation : 1 h

Récolte des cellules :
- Ôter le milieu, laver 2 fois avec PBS (tampon phosphate salin) ;
- Ajouter 500 µl par puits d'EDTA-NaOH (Acide éthylène diamine tétraacétique (EDTA) 0,02% + NaOH 200 µM), laisser reposer 10-15 minutes, puis détacher les cellules à l'aide d'un grattoir en caoutchouc et les transférer dans des tubes en verre à extraction ;
- Ajouter 10 µl d'HCl 20 mM, soniquer brièvement, puis transférer 10 µl de suspension dans des tubes Eppendorf de 1.5 ml pour le dosage des protéines ;
- Dans les tubes en verre, ajouter 500 µl de BHT (hydroxytoluène butylé) 200 µM/éthanol, 20 µl d'acétate de rétinyle 20 µM (standard interne), puis 4 ml d'hexane ;
- Vortexer vigoureusement 30 sec, puis centrifuger les tubes (900 g, 5 min) ;
- Transférer le surnageant dans des tubes en verre sans bouchon, évaporer à sec sous azote ;
- Reconstituer dans 200 µl de méthanol, puis transférer dans des flacons pour HPLC (chromatographie liquide haute performance).

Analyse des rétinoïdes par HPLC :
- chaîne HPLC Agilent 1100 avec pompe quaternaire et détecteur DAD
- colonne Macherey-Nagel Nucleodur C₁₈ pyramid 3 µm
- phase mobile :
   ∘ 0 - 6 min : méthanol 100%
   ∘ 6 - 8 min : gradient linéaire vers méthanol/THF (4:1)
   ∘ 8 - 18 min : méthanol/THF (4:1)
   ∘ 18 - 20 min : gradient linéaire vers méthanol 100%
   ∘ 20 - 30 min : méthanol 100%
- détection : 325 nm (rétinol, rétinyl esters); 383 nm (rétinal).

### Résultats :

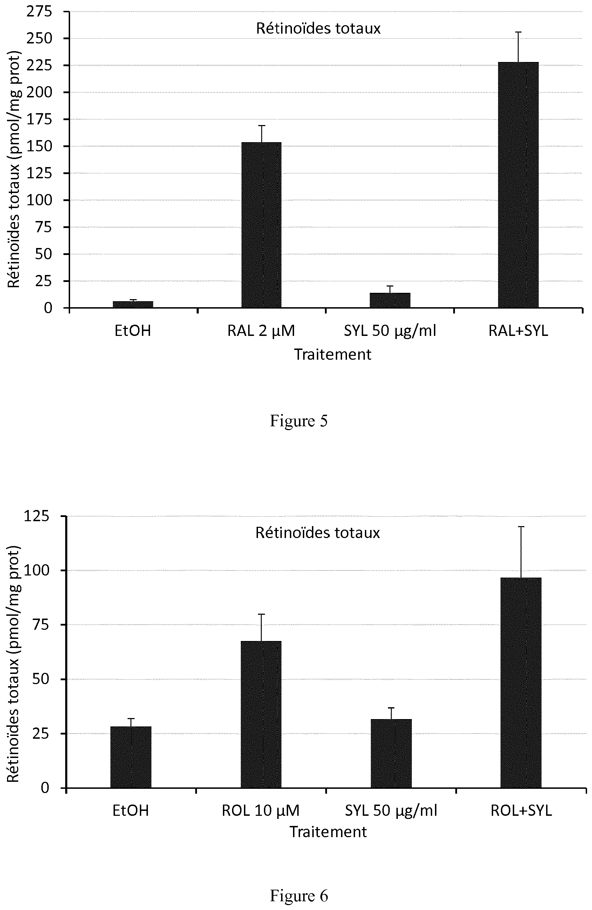

### Les résultats obtenus sont présentés sur les Figures 5 et 6.

### Conclusion :

L'extrait de *Silybum marianum* ne permet pas à lui seul d'induire une production significative de rétinoïdes.

En revanche, les cellules de la peau fabriquent significativement plus de rétinoïdes en présence de l'association d'un extrait de *Silybum marianum* avec du rétinaldhéyde ou du rétinol qu'en présence du rétinaldéhyde ou du rétinol seul démontrant l'effet synergique de l'association selon l'invention.

## Revendications

1. Association comprenant un rétinoïde et un extrait de *Silybum marianum* (L.) Gaertn., **caractérisée en ce que** le rétinoïde est le rétinaldéhyde et l'extrait de *Silybum marianum* (L.) Gaertn. est un extrait d'akènes de *Silybum marianum* (L.) Gaertn. comprenant moins de 0,2% en poids de silymarine par rapport au poids de l'extrait sec.

2. Association selon la revendication 1, **caractérisée en ce que** l'extrait de *Silybum marianum* (L.) Gaertn. est un extrait d'akènes de *Silybum marianum* (L.) Gaertn. comprenant moins de 0,1% en poids de silymarine par rapport au poids de l'extrait sec.

3. Association selon la revendication 1 ou 2, **caractérisée en ce que** l'extrait de *Silybum marianum* (L.) Gaertn. contient entre 0,5% et 2,5% en poids, notamment entre 1,0% et 2,0% en poids, par exemple environ 1,5% en poids de béta-sitostérol par rapport au poids de l'extrait sec, avec avantageusement un rapport massique silymarine/ béta-sitostérol inférieur à 0,4, notamment inférieur à 0,07, et notamment entre 2 et 7% en poids, en particulier entre 3 et 6% en poids, par exemple entre 3 et 5% en poids de stérols par rapport au poids de l'extrait sec.

4. Association selon l'une quelconque des revendications 1 à 3, **caractérisée en ce que** l'extrait de *Silybum marianum* (L.) Gaertn. contient entre 3% et 15% en poids, notamment entre 4% et 10% en poids, en particulier entre 4% et 6% en poids, par exemple environ 5% en poids d'acide linoléique libre par rapport au poids de l'extrait sec, et notamment entre 10% et 50%, en particulier entre 10% et 30% en poids, en particulier entre 15% et 25% en poids d'acides gras libres par rapport au poids de l'extrait sec.

5. Association selon l'une quelconque des revendications 1 à 4, **caractérisée en ce que** l'extrait de *Silybum marianum* (L.) Gaertn. comprend entre 0,01% et 0,5% en poids, notamment entre 0,05% et 0,2% en poids, par exemple environ 0,1% en poids de tocophérols par rapport au poids de l'extrait sec, avec avantageusement un rapport massique silymarine/tocophérols inférieur à 1, notamment inférieur à 0,1.

6. Association selon l'une quelconque des revendications 1 à 5, pour son utilisation comme médicament.

7. Association selon l'une quelconque des revendications 1 à 5, pour son utilisation dans le traitement de l'acné, de la séborrhée, de la rosacée et/ou de la dermite séborrhéique.

8. Composition dermatologique ou dermo-cosmétique comprenant au moins une association selon l'une quelconque des revendications 1 à 5 en combinaison avec au moins un excipient dermatologiquement ou dermo-cosmétiquement acceptable.

9. Composition dermatologique ou dermo-cosmétique selon la revendication 8, **caractérisée en ce qu'**elle comprend 0,01 à 10% en poids, de préférence 0,05 à 5% en poids, d'un extrait de *Silybum marianum* (L.) Gaertn., en poids d'extrait sec par rapport au poids total de la composition.

10. Composition dermatologique ou dermo-cosmétique selon l'une quelconque des revendications 8 et 9, **caractérisée en ce qu'**elle comprend 0,001 à 5% en poids, de préférence 0,01 à 1% en poids, du rétinoïde par rapport au poids total de la composition.

11. Composition dermatologique ou dermo-cosmétique selon l'une quelconque des revendications 8 à 10, **caractérisée en ce qu'**elle est destinée à une application topique, en particulier sur la peau.

12. Composition dermatologique ou dermo-cosmétique selon l'une quelconque des revendications 8 à 11, pour son utilisation dans le traitement de l'acné, de la séborrhée, de la rosacée et/ou de la dermite séborrhéique.

## Patentansprüche

1. Kombination, die ein Retinoid und einen Extrakt aus *Silybum marianum* (L.) Gaertn. umfasst, **dadurch gekennzeichnet, dass** das Retinoid Retinaldehyd ist und der Extrakt aus *Silybum marianum* (L.) Gaertn. ein Achänenextrakt aus *Silybum marianum* (L.) Gaertn. ist, das weniger als 0,2 Gew.-% Silymarin, bezogen auf das Gewicht des Trockenextrakts, umfasst.

2. Kombination nach Anspruch 1, **dadurch gekennzeichnet, dass** der Extrakt aus *Silybum marianum* (L.) Gaertn. ein Achänenextrakt aus *Silybum marianum* (L.) Gaertn. ist, der weniger als 0,1 Gew.-% Silymarin, bezogen auf das Gewicht des Trockenextrakts, umfasst.

3. Kombination nach Anspruch 1 oder 2, **dadurch gekennzeichnet, dass** der Extrakt aus *Silybum marianum* (L.) Gaertn. zwischen 0,5 Gew.-% und 2,5 Gew.-%, insbesondere zwischen 1,0 Gew.-% und 2,0 Gew.-%, zum Beispiel ungefähr 1,5 Gew.-% Beta-Sitosterol, bezogen auf das Gewicht des Trockenextrakts, enthält, wobei ein Silymarin/Beta-Sitosterol-Massenverhältnis vorzugsweise weniger als 0,4, insbesondere weniger als 0,07 und insbesondere zwischen 2 und 7 Gew.-%, vor allem zwischen 3 und 6 Gew.-%, zum Beispiel zwischen 3 und 5 Gew.-% Sterole, bezogen auf das Gewicht des Trockenextrakts, beträgt.

4. Kombination nach einem der Ansprüche 1 bis 3, **dadurch gekennzeichnet, dass** der Extrakt aus *Silybum marianum* (L.) Gaertn. zwischen 3 Gew.-% und 15 Gew.-%, insbesondere zwischen 4 Gew.-% und 10 Gew.-%, vor allem zwischen 4 Gew.-% und 6 Gew.-%, zum Beispiel ungefähr 5 Gew.-% freie Linolsäure, bezogen auf das Gewicht des Trockenextrakts, und insbesondere zwischen 10 Gew.-% und 50 Gew.-%, vor allem zwischen 10 Gew.-% und 30 Gew.-%, vor allem zwischen 15 Gew.-% und 25 Gew.-% freie Fettsäuren, bezogen auf das Gewicht des Trockenextrakts, enthält.

5. Kombination nach einem der Ansprüche 1 bis 4, **dadurch gekennzeichnet, dass** der Extrakt aus *Silybum marianum* (L.) Gaertn. zwischen 0,01 Gew.-% und 0,5 Gew.-%, insbesondere zwischen 0,05 Gew.-% und 0,2 Gew.-%, zum Beispiel ungefähr 0,1 Gew.-% Tocopherole, bezogen auf das Gewicht des Trockenextrakts, umfasst, wobei ein Silymarin/Tocopherole-Massenverhältnis vorzugsweise weniger als 1, insbesondere weniger als 0,1 beträgt.

6. Kombination nach einem der Ansprüche 1 bis 5, zur Verwendung als Medikament.

7. Kombination nach einem der Ansprüche 1 bis 5, zur Verwendung bei der Behandlung von Akne, Seborrhoe, Rosacea und/oder seborrhoischer Dermatitis.

8. Dermatologische oder dermokosmetische Zusammensetzung, umfassend mindestens eine Kombination nach einem der Ansprüche 1 bis 5 in Kombination mit mindestens einem dermatologisch oder dermokosmetisch verträglichen Hilfsstoff.

9. Dermatologische oder dermokosmetische Zusammensetzung nach Anspruch 8, **dadurch gekennzeichnet, dass** sie 0,01 bis 10 Gew.-%, vorzugsweise 0,05 bis 5 Gew.-%, eines Extrakts aus *Silybum marianum* (L.) Gaertn., bezogen auf das Gesamtgewicht der Zusammensetzung, umfasst.

10. Dermatologische oder dermokosmetische Zusammensetzung nach einem der Ansprüche 8 und 9, **dadurch gekennzeichnet, dass** sie 0,001 bis 5 Gew.-%, vorzugsweise 0,01 bis 1 Gew.-%, des Retinoids, bezogen auf das Gesamtgewicht der Zusammensetzung, umfasst.

11. Dermatologische oder dermokosmetische Zusammensetzung nach einem der Ansprüche 8 bis 10, **dadurch gekennzeichnet, dass** sie zur topischen Anwendung, vor allem auf der Haut, bestimmt ist.

12. Dermatologische oder dermokosmetische Zusammensetzung nach einem der Ansprüche 8 bis 11, zur Verwendung bei der Behandlung von Akne, Seborrhoe, Rosacea und/oder seborrhoischer Dermatitis.

## Claims

1. A combination comprising a retinoid and an extract of *Silybum marianum* (L.) Gaertn., **characterized in that** the retinoid is retinaldehyde and the extract of *Silybum marianum* (L.) Gaertn. is an extract of achenes of *Silybum marianum* (L.) Gaertn. comprising less than 0.2% by weight of silymarin based on the weight of the dry extract.

2. The combination according to claim 1, **characterized in that** the extract of *Silybum marianum* (L.) Gaertn. is an achene extract of *Silybum marianum* (L.) Gaertn. comprising less than 0.1% by weight of silymarin based on the weight of the dry extract.

3. The combination according to claim 1 or 2, **characterized in that** the extract of *Silybum marianum* (L.) Gaertn. contains between 0.5% and 2.5% by weight, in particular between 1.0% and 2.0% by weight, for example about 1.5% by weight of beta-sitosterol based on the weight of the dry extract, advantageously with a silymarin/beta-sitosterol weight ratio of less than 0.4, in particular less than 0.07, and in particular between 2 and 7% by weight, in particular between 3 and 6% by weight, for example between 3 and 5% by weight of sterols based on the weight of the dry extract.

4. The combination according to any one of claims 1 to 3, **characterized in that** the extract of *Silybum marianum* (L.) Gaertn. contains between 3% and 15% by weight, in particular between 4% and 10% by weight, in particular between 4% and 6% by weight, for example about 5% by weight of free linoleic acid based on the weight of the dry extract, and in particular between 10% and 50%, in particular between 10% and 30% by weight, in particular between 15% and 25% by weight of free fatty acids based on the weight of the dry extract.

5. The combination according to any one of claims 1 to 4, **characterized in that** the extract of *Silybum marianum* (L.) Gaertn. comprises between 0.01% and 0.5% by weight, in particular between 0.05% and 0.2% by weight, for example about 0.1% by weight of tocopherols based on the weight of the dry extract, with advantageously a silymarin/tocopherols weight ratio of less than 1, in particular less than 0.1.

6. The combination according to any one of claims 1 to 5, for use as a medicinal product.

7. The combination according to any one of claims 1 to 5, for use in the treatment of acne, seborrhea, rosacea and/or seborrheic dermatitis.

8. A dermatological or dermocosmetic composition comprising at least one combination according to any one of claims 1 to 5 in combination with at least one dermatologically or dermocosmetically acceptable excipient.

9. The dermatological or dermocosmetic composition according to claim 8, **characterized in that** it comprises 0.01 to 10% by weight, preferably 0.05 to 5% by weight, of an extract of *Silybum marianum* (L.) Gaertn., by weight of dry extract based on the total weight of the composition.

10. The dermatological or dermocosmetic composition according to any one of claims 8 and 9, **characterized in that** it comprises 0.001 to 5% by weight, preferably 0.01 to 1% by weight, of the retinoid based on the total weight of the composition.

11. The dermatological or dermocosmetic composition according to any one of claims 8 to 10, **characterized in that** it is intended for topical application, in particular to the skin.

12. The dermatological or dermocosmetic composition according to any one of claims 8 to 11, for use in the treatment of acne, seborrhea, rosacea and/or seborrheic dermatitis.
